# EUROPÄISCHE PATENTSCHRIFT

(11) **EP 0 548 537 B1**
(45) Veröffentlichungstag und Bekanntmachung des Hinweises auf die Patenterteilung: **03.07.1996**
(21) Anmeldenummer: 92119599.6
(22) Anmeldetag: 17.11.1992
(51) Int. Cl.: A61M 1/14

(54) **Einrichtung zur Herstellung einer medizinischen Flüssigkeit**
Apparatus for the preparation of a medical liquid
Appareil pour la préparation d'un liquide à usage médical

(30) Priorität: 28.11.1991 DE 4139165
(43) Veröffentlichungstag der Anmeldung: 30.06.1993
(73) Patentinhaber: Fresenius AG, D-61350 Bad Homburg (DE)
(72) Erfinder: Polaschegg, Hans-Dietrich, Dr., W-6370 Oberursel 4 (DE); Walter, Claus, W-6380 Bad Homburg (DE)
(74) Vertreter: Fuchs, Luderschmidt & Partner Patentanwälte

(56) Entgegenhaltungen:
- EP-A- 0 439 793
- EP-A- 0 443 324
- DE-A- 4 113 032
- US-A- 4 739 492

## Beschreibung

Die Erfindung betrifft eine Einrichtung zur Herstellung einer medizinischen Flüssigkeit, insbesondere einer Dialysierflüssigkeit, aus einem pulverförmigen Konzentrat und Wasser. Sie weist eine Wasserquelle, eine erste Leitung von der Wasserquelle zu einem das pulverförmige Konzentrat enthaltenden Behälter, eine zweite Leitung, die sich von dem Behälter bis zu einem Mischpunkt erstreckt, der in eine von der Wasserquelle abgehende dritte Leitung gelegt ist, eine Konzentratfördereinrichtung, die in die zweite Leitung geschaltet ist, eine Flüssigkeitsfördereinrichtung in der dritten Leitung stromab des Mischpunkts, eine Meßzelle im Leitungssystem, eine Steuereinheit, die auf das Signal der Meßzelle hin die Konzentratfördereinrichtung in vorbestimmter Weise steuert, sowie ein Schutzsystem auf, das die Einrichtung bei falscher Zusammensetzung der Flüssigkeit abschaltet.

Es ist bekannt, medizinische Flüssigkeiten, insbesondere Flüssigkeiten für die Hämodialyse, aus einem oder mehreren flüssigen Konzentraten und Wasser herzustellen. Letzteres ist insbesondere der Fall bei bicarbonathaltigen Dialysierflüssigkeiten, da deren Bestandteile in konzentrierter Form nicht völlig in Lösung bleiben.

In der EP-A- 0 278 100 ist eine Einrichtung der eingangs erwähnten Art beschrieben, bei der die Dialysierflüssigkeit dadurch hergestellt wird, daß Wasser in einen ein pulverförmiges Konzentrat aufweisenden Behälter geleitet wird, in dem es bis zur Sättigungsgrenze die pulverförmigen Bestandteile löst. Das flüssige Salzkonzentrat wird dann weiter mit Wasser bis zu einer gewünschten Konzentration verdünnt. Hierzu wird das flüssige Konzentrat mit Wasser an einem Mischpunkt vermischt, woraufhin das erhaltene Gemisch stromab des Mischpunkts einer Leitfähigkeitszelle zugeführt wird. Das Signal der Leitfähigkeitszelle wird an eine Steuereinheit übermittelt, die das erhaltene Signal mit einem vorgegebenen Wert vergleicht und bei einer Abweichung vom Soll-Wert eine Konzentratfördereinrichtung, die üblicherweise als Konzentratpumpe ausgebildet ist, so steuert, daß sich eine bestimmte vorgegebene Leitfähigkeit stromab des Mischpunkts einstellt, d.h. die Dialysierflüssigkeit eine vorbestimmte Zusammensetzung aufweist.

Weiterhin weist eine solche Einrichtung ein Schutzsystem gegen eine falsche Zusammensetzung der Dialysierflüssigkeit auf, das in der abgehenden Leitung angeordnet ist und die Zusammensetzung der fertigen Dialysierflüssigkeit überwacht. Üblicherweise werden hierzu ebenfalls Leitfähigkeitsmeßzellen eingesetzt, die zum Schutz des Patienten vor Dialyse mit ungeeigneter Dialysierflüssigkeit (Richtlinien gemäß VDE 750, Teil 206 oder Teil 213) unabhängig von der Leitfähigkeitszelle sein müssen, die zur Regelung der Zusammensetzung der Dialysierflüssigkeit eingesetzt wird.

Erfolgt nun die Regelung der Zusammensetzung der Dialysierflüssigkeit mit einem Sensor, der die Endzusammensetzung der Dialysierflüssigkeit bestimmt, wie dies in der vorstehend genannten EP 0 278 100 dargestellt ist, und ist weiterhin das übliche Schutzsystem stromab dieses Sensors in Form einer weiteren Leitfähigkeitszelle vorgesehen, so kann zum Beispiel nicht erkannt werden, wenn ein Konzentrat ähnlicher Leitfähigkeit eingesetzt wird, das eine Gefahr für den Patienten bedeutet. Typisch ist die Verwechslungsmöglichkeit von HD-Konzentrat mit Bleichlauge oder darauf aufbauenden Desinfektionsmitteln im wesentlichen gleicher Ausgangsleitfähigkeit. Insofern besitzen LF-geregelte Geräte einen zweiten Sensor, dem ein anderes Meßprinzip zugrundeliegt, beispielsweise einen pH-Sensor als zusätzliches Schutzsystem. Letzterer ist jedoch nicht zuverlässig, da er im Betrieb weder kalibriert noch geprüft werden kann. Auf entsprechende Unfallberichte der FDA wird verwiesen.

Andererseits gibt es auch Hämodialysegeräte, die mit volumetrischen Mischsystemen arbeiten, bei denen flüssiges Konzentrat und Wasser in einem vorbestimmten Verhältnis miteinander gemischt werden. Solche Systeme haben sicherheitstechnische Vorteile, können jedoch nicht ohne weiteres vom Betrieb mit einem flüssigen Konzentrat, das vom Hersteller mit einer vorbestimmten Zusammensetzung geliefert wird, auf ein aus pulverförmigem Konzentrat und Wasser hergestelltes flüssiges Konzentrat umgestellt werden, da dieses Konzentrat in der Regel eine andere Zusammensetzung als das eingangs genannte Konzentrat aufweist.

Weiterhin wurde festgestellt, daß bei der Herstellung von Bicarbonatkonzentrat durch Vermischen mit Wasser erhebliche Mengen CO₂ entweichen, so daß hierdurch das Gleichgewicht von Bicarbonat zu Carbonat verschoben wird.

Schließlich wird bei dem gemäß der EP-A- 0 278 100 eingesetzten Behälter, der starr ausgeführt ist, eine erhebliche Menge Wasser vergeudet, wenn dieser Behälter von dem ursprünglich vorhandenen pulverförmigen Konzentrat entleert ist.

Der Erfindung liegt daher die Aufgabe zugrunde, eine Vorrichtung der eingangs erwähnten Art zur Verfügung zu stellen, die gegenüber verschiedenen Konzentraten mit gleicher Anfangsleitfähigkeit sicher ist.

Gemäß einer weiteren Aufgabe soll die Entgasung von CO₂ beim Vermischen weitgehend vermindert werden, sofern Bicarbonat als Ausgangspulver eingesetzt wird.

Schließlich soll gemäß einer dritten Aufgabe der das pulverförmige Konzentrat enthaltende Behälter am Ende der Mischoperation möglichst wenig Wasser aufweisen.

Die der Erfindung zugrundeliegende Aufgabe wird dadurch gelöst, daß die Meßzelle in der vom Behälter zum Mischpunkt abgehenden Leitung angeordnet ist, in der das flüssige Konzentrat zum Vermischen mit Wasser vorliegt.

Überraschenderweise wurde hierdurch festgestellt, daß auch beim Einsatz von Konzentraten mit gleicher Anfangsleitfähigkeit, jedoch unterschiedlicher chemischer Zusammensetzung, die Sicherheit der Gesamtvorrichtung gegeben ist. Das Schutzsystem stromab des Mischpunkts kann nämlich eine falsche Zusammensetzung aufgrund unterschiedlicher Leitfähigkeitswerte der endgültig hergestellten Flüssigkeiten erkennen. Die erfindungsgemäße Einrichtung weist nämlich zum einen eine für die Mischoperation dienende Meßzelle in Form einer Leitfähigkeitszelle in der Konzentratleitung auf, die den Istwert der im Behälter erzeugten Konzentratflüssigkeit bestimmt. Dieser Istwert wird in einer Steuereinheit mit einem vorgegebenen Sollwert, der die die Zusammensetzung der fertigen Flüssigkeit darstellen soll, verglichen. Aufgrund des Vergleichs wird dann die Flüssigkeitsfördereinrichtung, die üblicherweise eine Konzentratpumpe ist, entsprechend gesteuert. Dies geschieht in Abhängigkeit von der Förderrate, mit der Wasser dem Mischpunkt zugeführt wird. Auch dieser Wert wird in die Steuereinheit eingegeben, so daß hier das Verhältnis von Konzentratmenge/Wasser vorgegeben ist.

Demgegenüber ist die als Schutzsystem eingesetzte Leitfähigkeitszelle stromab des Mischpunkts angeordnet und überwacht dort die fertige Flüssigkeit.

Die Wirkungsweise der erfindungsgemäßen Vorrichtung soll an einem Beispiel erläutert werden:
Eine typische fertige Natriumbicarbonat-Lösung für eine Dialysierflüssigkeit weist eine Leitfähigkeit von 2,45 mS/cm auf und hat eine Konzentration von 0,035 m/l. Sie wird hergestellt durch 21,2-fache Verdünnung aus einem Konzentrat, das 0,743 m/l Bicarbonat enthält. Diese Lösung weist eine Leitfähigkeit von 37 mS/cm auf. Alternativ können andere Ausgangskonzentrationen (z.B. 1 molar, 0,5 molar) und damit andere Verdünnungen eingesetzt werden.

Nachdem das Mischverhältnis von 21,2 fest eingestellt ist, muß der Steuerungssensor auf eine Leitfähigkeit von 37 mS/cm eingestellt werden. Wird nun das Konzentrat verwechselt, wobei anstelle von Bicarbonat Natriumchlorid eingesetzt wird, so weist ein Natriumchlorid-Konzentrat mit einer Leitfähigkeit von 37 mS/cm eine Konzentration von 0,435 m/l auf. Nach Verdünnung mit dem vorgegebenen Verhältnis von 21,2:1 wird jedoch eine Flüssigkeit erhalten, die nur eine Leitfähigkeit von 2,05 mS/cm aufweist. Nachdem das Schutzsystem auf einen LF-Wert von 2,45 mS/cm mit üblichen Fehlergrenzen von ± 5% eingestellt ist, kann dieser deutliche Fehler sicher erkannt und die gesamte Vorrichtung in den sicheren Zustand durch Abschalten überführt werden.

Die erfindungsgemäße Vorrichtung weist vorteilhafterweise einen Temperatursensor auf, der mit dem das flüssige Konzentrat aufweisenden Behälter verbunden ist, um den temperaturabhängigen Leitfähigkeitswert entsprechend zu korrigieren.

Die Leitfähigkeitswerte der Konzentrate und der fertigen Dialysierflüssigkeit auf der Basis von Bicarbonat sind in Standardtabellenwerken abgelegt, beispielsweise in CRC Handbook of Chemistry and Physics und können in der Steuereinheit abgelegt sein. Hieraus können durch Korrelation die gewünschten Konzentrationen des Konzentrats und der endgültigen Flüssigkeit in Abhängigkeit von der Förderrate des zugeführten Wassers bestimmt werden. Üblicherweise ist die gesamte Leitfähigkeitstabelle eines flüssigen Konzentratsystems in der Steuereinheit abgelegt und dient dort als Algorithmus zur Festlegung der Förderrate der Konzentratpumpe.

Vorteilhafterweise kann die Leitfähigkeitsmeßzelle in einem Konzentratansaugrohr integriert sein, wie es beispielsweise in der DE-A- 37 34 880 beschrieben ist, worauf hiermit aus Offenbarungszwecken Bezug genommen wird. Das System zur Herstellung von Konzentrat aus Pulver und Wasser verfügt dann über eine im wesentlichen zylindrische Aufnahme für das besagte Ansaugrohr. Der besondere Vorteil dieser Ausführungsform liegt in der Möglichkeit der wahlweisen Verwendung von flüssigem und pulverförmigem Konzentrat. Da die im Ansaugrohr integrierte Leitfähigkeitszelle nur zur Unterscheidung von Konzentraten dient, ist sie in einer praktischen Ausführungsform ohne Temperatursensor ausgebildet. Zur Erzielung einer genauen Konzentration ist jedoch - wie vorstehend erläutert - eine Temperaturkompensation vorzunehmen. Insofern wird in einer weiteren speziellen Ausführungsform die Temperatur des zur Herstellung des Konzentrats vorgesehenen Wassers geregelt und die Temperatur des Konzentrats gemessen. In einem solchen Fall verfügt man daher über eine redundante Messung von Konzentratparametern.

Wie bereits vorstehend erläutert, verfügt das Mischsystem eines solchen Gerätes, das neben der Herstellung von Dialysierflüssigkeiten auch zur Herstellung von Infusionslösungen eingesetzt werden kann, über eine Steuereinrichtung, die ein Temperatursignal des Konzentrat-/Temperatursensors und/oder ein Leitfähigkeitssignal des Leitfähigkeitssensors des Ansaugrohrs, ein Sollkonzentrationssignal und ein Fluß-Sollsignal von einer Einstelleinrichtung erhält. Die Regeleinrichtung regelt daraus mit Hilfe des vorgegebenen Algorithmus die Sollfördermenge für die Konzentratpumpe.

Gemäß einem weiteren Erfindungsgedanken wurde überraschend erkannt, daß die Verwendung von entgastem Wasser, also Wasser, das von der in ihm gelösten Luft befreit ist, die Qualität des flüssigen Bicarbonatkonzentrats wesentlich verbessert. Nimmt man nämlich luftgesättigtes Wasser zur Herstellung von flüssigen Bicarbonatkonzentraten, so kommt es zu einem starken Entweichen von CO₂ und damit zu einer erheblichen Verschiebung des Bicarbonat/Carbonat-Gleichgewichts. Insofern mußte bisher eine erhebliche Menge an Säure zugesetzt werden, um den pH-Wert in den sauren Bereich zu verschieben. Durch den Einsatz von entgastem Wasser kann der sonst übliche Zusatz von Säure aus einem Säurekonzentrat, das als zweites Konzentrat zugemischt wird und das Gleichgewicht in Richtung Bicarbonat verschieben soll, stark verringert werden.

Einheiten zur Entgasung von Dialysierflüssigkeit sind bei Hämodialysegeräten üblich. Daher kann auf diese Entgasungseinheiten zurückgegriffen werden, wobei jedoch das flüssige Bicarbonatkonzentrat nicht stromauf der Entgasungseinheit, sondern vielmehr stromab der Entgasungseinheit zugeführt wird. Eine Einrichtung zur Entgasung von Wasser besteht üblicherweise aus einer Unterdruckstrecke, mit der die in Wasser gelöste Luft ausgetrieben wird. Vorteilhafterweise erfolgt die Förderung des Wassers im Rezirkulationsbetrieb, wobei einem Reservoir zugeführtes Frischwasser im Kreislauf durch die Entgasungsstrecke wieder dem Reservoir zurückgeführt wird, in dem eine Niveauregelung erfolgt.

Gemäß einem weiteren übergeordneten Erfindungsgedanken ist der das Pulver enthaltende Behälter in Form eines zusammenfaltbaren Beutels ausgeführt, der sich während der Verwendung durch Aufnahme und Abgabe von Wasser bzw. Flüssigkeit ausdehnt und zusammenzieht, am Ende der Behandlung nur noch ein geringes Füllgewicht aufweist und somit ein geringes Abfallvolumen verursacht.

Dieser Beutel weist vorteilhafterweise einen obenliegenden Zulaufstutzen und einen untenliegenden Ablaufstutzen auf, an denen jeweils ein Konnektorstück befestigt ist, die mit einem an der Vorrichtung angeordneten komplementären Konnektorstück verbunden werden können.

Gemäß einer ersten Ausführungsform ist der Beutel so angeordnet, daß die unter Vermischen mit Wasser hergestellte Flüssigkeit unter Einwirkung der Schwerkraft in einen Konzentratvorlaufbehälter fließt, an den die Konzentratpumpe angeschlossen ist.

In einer weiteren Ausführungsform wird der das Pulver enthaltende Behälter diskontinuierlich mit Wasser versorgt, wobei zunächst Wasser über einen vorbestimmten Zeitraum durch ein geöffnetes Zulaufventil zugeführt wird. Insofern füllt sich der Behälter mit einem bestimmten Volumenteil Wasser. Daraufhin läßt man dieses zugeführte Wasser auf das pulverförmige Salz solange einwirken, bis eine gesättigte Lösung erhalten wird. Nach dieser vorbestimmten Verweilzeit wird ein Ablaufventil betätigt, so daß die gesättigte Lösung in einen Konzentratvorlaufbehälter fließen kann. Dieser Vorgang kann entweder während einer vorbestimmten Öffnungsperiode des Ablaufventils stattfinden oder er kann mit Hilfe einer am Vorlaufbehälter befindlichen Niveausensoreinrichtung geregelt werden, die zwischen einem oberen und einem unteren Niveauwert pendelt. Letztere Ausführungsform ist bevorzugt. Deshalb wird das Ablaufventil erst dann wieder geöffnet, wenn das Niveau im Vorlaufbehälter auf einen unteren Grenzwert abgesunken ist. Hierauf beginnt wiederum die Füllung des Vorlaufbehälters. Nachdem das Volumen zwischen diesen beiden Niveauwerten im Vorlaufbehälter bekannt ist und auch das zugeführte Volumen Frischwasser vorbestimmt bzw. durch ein Volumen-/Gewichtsmeßgerät bestimmbar ist, kann zu gegebener Zeit das Einlaufventil zur Auffüllung des Beutels betätigt werden.

Wie bereits festgestellt, ist gemäß einer bevorzugten Ausführungsform das Einlaufventil mit einem Volumen-/Gewichtsmeßgerät verbunden, so daß hierdurch die zugeführte Wassermenge bestimmt werden kann.

Wird beispielsweise ein Wegaufnehmer verwendet, der die Aufblähung des Beutels bei der Zuführung von Wasser bestimmt, so ist die Auslenkung dieses Wegaufnehmers ein Maß für die zugeführte Flüssigkeitsmenge. Fällt also die im Beutel enthaltene Flüssigkeitsmenge durch Öffnen des Ablaufventils ab, so verändert sich die vom Wegaufnehmer zurückgelegte Wegstrecke, die mit zunehmender Auflösung des Salzes im Beutel einen geringeren Auslenkungswert annimmt. Auf diese Weise läßt sich die endgültige Auflösung des Salzes bestimmen, und zwar beispielsweise dadurch, daß die ersten Werte des Wegaufnehmers (jeweils ungefüllt mit Pulver und erstmals aufgefüllt mit Wasser) als Bezugswerte dienen. Ist beispielsweise am Ende der Mischoperation der erste Differenzwert vorhanden, so läßt dies auf eine völlige Auflösung des ursprünglich vorhandenen Salzes schließen.

Gemäß einer weiteren Ausführungsform verringert sich die Wegstrecke nach dem Zuführen des Wassers um einen bestimmten Betrag, der mit dem Volumen des aufgelösten Salzes korreliert ist. Dabei wird vorausgesetzt, daß sich im wesentlichen die Dichte des Salzkonzentrats nicht ändert. Andererseits kann jedoch aber auch ein Korrekturfaktor in dieser Hinsicht berücksichtigt werden. Wird nunmehr die zeitliche Abhängigkeit dieser Abnahme im Rechner registriert, so kann aus dieser Abnahme ohne weiteres das endgültige Aufbrauchen des Salzes errechnet werden. Dieser Rechner kann also zu einer vorbestimmten Zeit die gesamte Mischoperation kurz vor dem Aufbrauchen des Salzes abbrechen und den gesamten Beutel bis auf die restlichen Salzbestandteile entleeren.

Gemäß einer weiteren Ausführungsform wird der Meßsensor zur Überwachung der Zusammensetzung benutzt. Folgt ein krasser Abfall der Zusammensetzung, beispielsweise von 30 % und mehr, so schaltet die gesamte Anordnung in den sicheren Zustand. Hierbei wird der gesamte Beutel durch die Konzentratpumpe in eine Bypass-Leitung gepumpt.

Andererseits können auch Lösungen definierter Zusammensetzungen aus den pulverförmigen Massen hergestellt werden. Hierzu wird das Wasserzulaufventil solange geöffnet, bis eine vorbestimmte Wassermenge, die zur vollständigen Lösung des im Beutel enthaltenen Pulvers ausreicht, eingeströmt ist. Diese Wassermenge kann, wie vorstehend erwähnt, gesteuert oder aber auch geregelt mit Hilfe eines Volumen-/Mengenmeßgeräts zugeführt werden. Anschließend wird solange gewartet, bis eine vollständige Lösung stattgefunden hat. Vorteilhafterweise kann der mit dem Pulver und Wasser versehene Beutel durch mechanische Bewegung (Schütteln oder Quetschen des Beutels) oder durch Rezirkulation der Flüssigkeit und Erhöhung der Wassertemperatur beschleunigt werden.

Die erfindungsgemäße Einrichtung läßt sich nicht nur zur Herstellung von Hämodialysekonzentrat oder Hämodialysierflüssigkeit verwendet. Sie kann vielmehr auch zur Herstellung einer Substitutionslösung für die Hämofiltration oder einer Infusionslösung eingesetzt werden.

Es können auch mehrere der genannten Einrichtungen kombiniert werden, so daß ein Multimischsystem erhalten wird, mit dem mehrere Komponenten enthaltende Lösungen hergestellt werden können. Eine solche Vorgehensweise ist insbesondere dann angebracht, wenn Lösungen aus mehreren Salzen, die eine unterschiedliche Löslichkeit in Wasser aufweisen, hergestellt werden sollen. Hierzu wird dann je eine Einrichtung für ein Salz zu dessen schrittweise verlaufenden Auflösung eingesetzt, wobei die Summe der Einrichtungen dann das oben genannte Multimischsystem ergibt.

Vorteile, Ausführungsformen und Merkmale sind aus der nachfolgenden Beschreibung von Ausführungsbeispielen ersichtlich.

Es zeigen
- Figur 1: eine schematische Skizze der erfindungsgemäßen Einrichtung,
- Figur 2: einen schematischen Ausschnitt von Figur 1, der die Wasserquelle mit dem Entgasungsbereich darstellt,
- Figur 3: eine schematische Ansicht einer weiteren Ausführungsform der Erfindung, in der eine erste Füll-/Entleerungsoperation gezeigt ist und
- Figur 4: einen schematischen Ausschnitt einer dritten Ausführungsform der Erfindung, die sich auf einen Wegaufnehmer zur Bestimmung des Füllzustandes des erfindungsgemäßen Beutels bezieht.

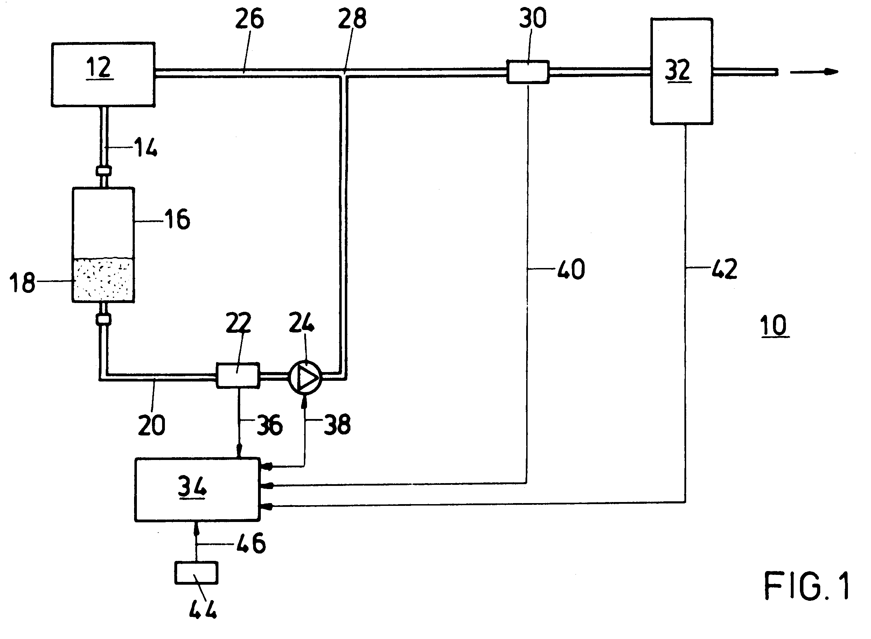

In Figur 1 ist schematisch eine Einrichtung 10 zur Herstellung von medizinischen Flüssigkeiten aus Wasser und einem Salzkonzentrat gezeigt. Die Einrichtung 10 weist eine Wasserquelle 12 auf, von der eine erste Leitung 14 abgeht, die mit einem Behälter 16 verbunden ist, der ein Salzkonzentrat 18 in Pulverform aufweist.

Der Behälter 16 ist vorteilhafterweise in Form eines Beutels ausgebildet.

Von dem Behälter 16 geht eine zweite Leitung 20 ab, in die eine erste Meßzelle 22 eingeschaltet ist, die vorteilhafterweise als Leitfähigkeitssensor ausgebildet ist.

Stromab der ersten Meßzelle 22 ist eine Konzentratpumpe 24 in die zweite Leitung 20 eingeschaltet, die als Flüssigkeitsfördereinrichtung dient. Diese Konzentratpumpe 24 kann auch stromauf der ersten Meßzelle 22 angeordnet sein.

Von der Wasserquelle 12 geht weiterhin eine dritte Leitung 26 ab, in die am Mischpunkt 28 die zweite Leitung 20 mündet. Stromab des Mischpunkts 28 ist eine zweite Meßzelle 30 vorteilhafterweise in Form eines Leitfähigkeitssensors zu Überwachungszwecken eingeschaltet. Hieran schließt sich eine Förder- oder Bilanziereinrichtung 32 am Ende der dritten Leitung 26 an, mit der das fertige flüssige Gemisch gefördert bzw. bilanziert wird. Hierzu kommt entweder eine zweite Pumpe oder aber eine Bilanzkammeranordnung in Betracht, wie sie beispielsweise bei dem Dialysegerät der Anmelderin mit der Bezeichnung A 2008 eingesetzt wird.

Weiterhin ist eine Steuer- und Regeleinrichtung 34 vorgesehen, die über Leitungen 36-42 mit der ersten Meßzelle 22, der Konzentratpumpe 24, der zweiten Meßzelle 30 und der Förder- und Bilanziereinrichtung 32 verbunden ist. Schließlich ist eine Eingabeeinheit 44 vorgesehen, die über die Leitung 46 mit der Steuer- und Regeleinrichtung 34 verbunden ist.

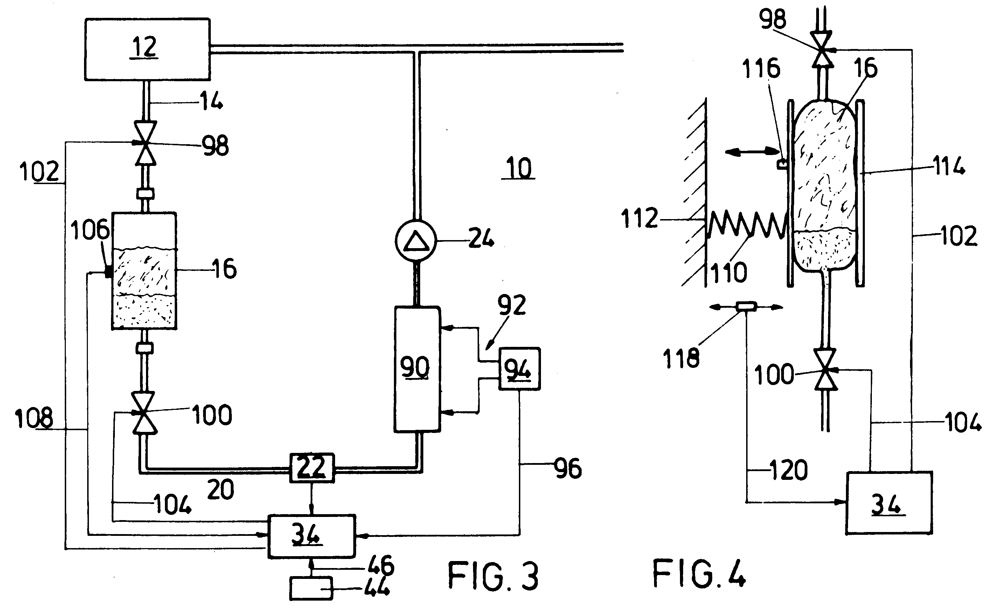

Aus Gründen der Vereinheitlichung ist die Steuerund Regeleinrichtung nicht mit den in Figuren 3 und 4 beschriebenen Einrichtungen zur Regelung des Zu- und Abflusses des Beutels 16 verbunden.

Die in Figur 1 gezeigte Einrichtung 10 wird folgendermaßen betrieben.

Aus der Wasserquelle 12 wird über die Leitung 14 Frischwasser zugeführt, in dem ein Vermischung und Auflösung des dort enthaltenen Salzes 18 erfolgt. Die erhaltene Konzentratlösung wird durch die Wirkung der Konzentratpumpe 24 durch die Leitung 20 dem Mischpunkt 28 zugeführt.

Dem Mischpunkt 28 wird weiterhin über die dritte Leitung 26 Wasser zugeführt, wo die Vermischung der beiden Komponenten zu der Endlösung mit vorbestimmter Zusammensetzung erfolgt. Dabei erfolgt die Förderung mit Wasser mit der Fördereinrichtung 32, deren Signale über die Signalleitung 42 an die Steuereinheit 34 in vorteilhafter Weise abgegeben wird, sofern diese nicht fest in ihrer Förderrate eingestellt ist. Letzterer Wert kann jedoch aber auch über die Eingabeeinheit 44 in die Steuereinheit 34 fest eingegeben sein.

Die Einheit 34 ist weiterhin mit der ersten Meßzelle 22 verbunden, die die aktuelle Zusammensetzung des flüssigen Konzentrats bestimmt und an die Einheit 34 übermittelt. Da diese Meßzelle 22 vorteilhafterweise als Leitfähigkeitssensor ausgebildet ist, wird ein Leitfähigkeitswert übermittelt, der nur in erster Näherung linear von der Konzentration (Mol Salz/Liter Wasser) abhängt.

Insofern ist vorteilhafterweise in der Einheit 34 eine Vielzahl von Leitfähigkeitsdaten abgelegt, die mit den jeweiligen Konzentrationswerten korrelieren. Nachdem die endgültige Lösung durch ihre molare Konzentration bestimmt ist, errechnet die Einheit 34 den Konzentrations-Ist-Wert der Konzentratlösung und vergleicht diesen mit einem zuvor über die Eingabeeinheit 44 eingegebenen Soll-Wert.

Die Einheit 34 ist weiterhin über die Leitung 38 mit der Konzentratpumpe 24 verbunden, die in ihrer Förderrate durch die Steuereinheit 34 verändert werden kann. Aufgrund des errechneten Vergleichsergebnisses wird die Konzentratpumpe 24 entsprechend geregelt, so daß dem Mischpunkt 28 die jeweils benötigte Konzentratmenge zu der von der Fördereinrichtung 32 geförderten Wassermenge zugefördert wird.

Demzufolge wird also die von der Pumpe 24 geförderte Konzentratflüssigkeit in einem vorbestimmten Leitfähigkeitswert eingestellt, der im wesentlichen bereits an der ersten Meßzelle 22 erhalten wird.

Die Überwachung erfolgt durch die zweite Meßzelle 30 in der dritten Leitung 26 stromab des Mischpunkts 28, die unabhängig von der ersten Meßzelle 22 die endgültige Zusammensetzung der Flüssigkeit bestimmt. Weicht der gemessene Leitfähigkeitswert vom vorgegebenen Wert um ± 5 % ab, so wird über die Leitung 40 die gesamte Einheit 34 stillgesetzt.

Die Einrichtung 10 gemäß Figur 1 ist - wie eingangs beschrieben - sicher, da beide Meßzellen 22 und 30 unabhängig voneinander und in anderen Konzentrationsbereichen arbeiten. Dies folgt daraus, daß Konzentrate unterschiedlicher Zusammensetzung mit gleicher Anfangs-, jedoch unterschiedlicher Endleitfähigkeit sicher auseinandergehalten werden können.

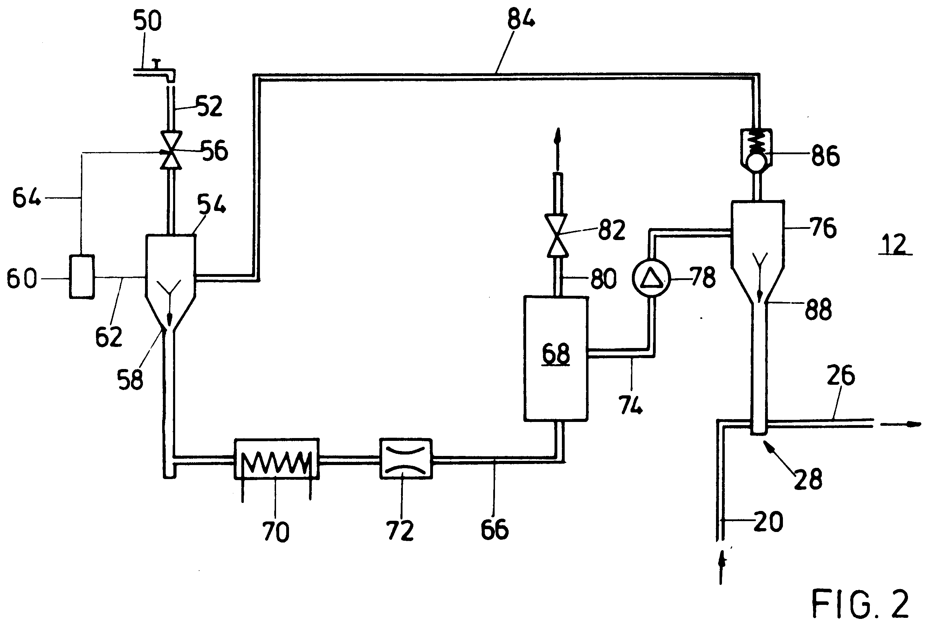

In Figur 2 ist eine spezielle Ausführungsform der Wasserquelle 12 gezeigt. Diese Wasserquelle 12 dient zur Entgasung von Frischwasser, wodurch - wie eingangs erläutert - ein erheblich günstigeres Entgasungsverhalten einer bicarbonathaltigen Dialysierflüssigkeit erzielt wird.

Die Wasserquelle 12 weist zunächst einen Frischwasseranschluß 50 auf, von dem eine Leitung 52 abgeht, die mit einem Wasserreservoir 54 verbunden ist. In die Leitung 52 ist ein Frischwasserventil 56 eingeschaltet. Das Wasserreservoir 54 weist eine Niveauregulierung 58 auf, die über die Steuereinheit 60 und die beiden Leitungen 62, 64 mit dem Frischwasserventil 56 zusammenwirkt.

Von dem Frischwasserreservoir 54 geht eine Leitung 66 ab, die in den Boden eines Entgasungsbehälters 68 mündet. In der Leitung 66 ist eine Heizung 70, mit der das Frischwasser auf eine vorbestimmte Temperatur gebracht wird, und ein Drosselventil 72 angeordnet.

Vom Entgasungsbehälter 68 geht eine weitere Leitung 74 ab, die in ein zweites Wasserreservoir 76 mündet und in die eine Umwälzpumpe 78 eingeschaltet ist, die zusammen mit dem Drosselventil 72 eine als Entgasungseinheit wirkende Unterdruckeinheit darstellt. Am oberen Ende des Entgasungsbehälters 68 geht eine Entgasungsleitung 80 ab, in die ein Entgasungsventil 82 eingeschaltet ist, das mit einer nicht gezeigten Unterdruckeinheit verbunden ist, um in regelmäßigen Zeitabständen die sich angesammelte, ausgegaste Luft zu entfernen.

Das Wasserreservoir 76 weist an seinem oberen Ende eine Rezirkulationsleitung 84 auf, die zum ersten Wasserreservoir 65 zurückgeführt ist und in die ein Rückschlagventil 86 eingeschaltet ist.

Des weiteren kann in dem zweiten Wasserreservoir 76, das sich vorteilhafterweise nach unten verjüngt, eine Schwimmeranordnung 88 vorgesehen sein, mit der eine gewisse Medientrennung für das - wie aus Figur 2 ersichtlich ist - oben einströmende Wasser und das über die Konzentratleitung 20 am unteren Ende einströmende flüssige Konzentrat vorgenommen werden kann. Dies ist insbesondere bei batch-weiser Herstellung von Dialysierflüssigkeit, wie es bei der von der Anmelderin in der Dialyseanordnung A 2008 eingesetzten Bilanzkammeranordnung der Fall ist, vorteilhaft. Demzufolge stellt der untere Teil des zweiten Wasserreservoirs 76 den Mischpunkt 28 gemäß Figur 1 dar. Von diesem unteren Teil ist die dritte Leitung 26 vom Mischpunkt 28 weg zum Verbraucher abgeführt, der die in Figur 1 dargestellte Förder- oder Bilanziereinheit 32 darstellen kann.

Die Wasserquelle gemäß Figur 2 arbeitet folgendermaßen:
Zunächst wird das Frischwasserventil 56 geöffnet und das erste Reservoir 54 solange gefüllt, bis die Niveauregeleinrichtung 58 über die Steuereinheit 60 das Ventil 56 schließt. Mit Hilfe der Pumpe 78 wird aus dem Reservoir das Frischwasser durch die Leitung 66, den Heizer 70 und das Drosselventil 72 in den Entgasungsbehälter 68 gepumpt. Dabei wird zwischen der Drossel 72 und dem Eingang der Unterdruckpumpe 78 ein solcher Unterdruck erzeugt, daß die im Frischwasser physikalisch gelöste Luft ausgetrieben wird. Diese Luft sammelt sich im Entgasungsbehälter 68 und kann, wie vorstehend erwähnt, durch die Entgasungseinheit 80 abgeschieden werden.

Durch die Wirkung der Pumpe 78 gelangt das entgaste Wasser in das zweite Wasserreservoir 76 und verläßt dieses nach dem Füllen des Reservoirs durch die Rezirkulationsleitung 84 zum ersten Wasserreservoir 54. In dem zweiten Wasserreservoir 76 erfolgt eine Vermischung mit der von unten zugeführten flüssigen Konzentratlösung, wobei das erhaltene Gemisch durch die Leitung 26 ausgetragen wird. Wie bereits vorstehend erwähnt, kann diese Vermischung kontinuierlich, sofern Flüssigkeit im Dauerbetrieb hergestellt wird, oder aber batch-weise erfolgen, sofern eine Bilanzkammeranordnung eingesetzt wird, wie sie beispielsweise in der DE-OS 28 38 414 beschrieben ist.

In Figur 3 ist eine schematische Ansicht einer weiteren Ausführungsform der erfindungsgemäßen Einrichtung 10 ersichtlich, mit der eine erste Füll- und Entleerungsoperation durchgeführt werden kann.

Diese Einrichtung 10 weist zusätzlich zu der in Figur 1 gezeigten Ausführungsform zwischen der ersten Meßzelle 22 und der Pumpe 24 einen Vorlaufbehälter 90 auf, an dem eine Niveausensoreinheit 92 angeordnet ist, wie dies durch die beiden Pfeile dargestellt ist, die den unteren bzw. den oberen Niveauwert darstellen. Diese Niveausensoreinheit 92 ist über eine Steuereinheit 94 sowie eine Leitung 96 mit der Steuer- und Regeleinheit 34 verbunden. Weiterhin ist in die Leitung 14 ein Einlaßventil 98 und in die Leitung 20 ein Auslaßventil 100 eingeschaltet, die über Steuerleitungen 102 bzw. 104 mit der Steuereinheit 34 verbunden sind. Schließlich ist ein Temperatursensor 106 an dem Behälter 16 vorgesehen, der über eine Leitung 108 mit der Steuereinheit 34 verbunden ist.

Die in Figur 3 gezeigte Einheit 10 wird folgendermaßen betrieben:
Das Einlaßventil 98 und das Auslaßventil 100 wird wechselweise betätigt, wobei das Auslaßventil 100 dann ausgeschaltet wird, wenn der obere Grenzwert des Niveausensors 92 erhalten wird. Desgleichen wird das Auslaßventil 100 dann geöffnet, wenn das Niveau auf den unteren Wert des Niveausensors 92 gefallen ist.

Nachdem üblicherweise das Innenvolumen des Vorlaufbehälters 90 bekannt ist, und auch die durch das Einlaßventil 98 zulaufende Wassermenge ebenfalls abgeschätzt werden kann, ist die Steuereinheit 34 in der Lage, das Einlaufventil 98 in Abhängigkeit von den Takten des Auslaßventils 100 zu öffnen bzw. zu schließen.

Im übrigen wird auf die weiteren Betriebsweisen verwiesen, die vorstehend bereits erläutert worden sind.

Schließlich kann es zweckmäßig sein, daß die in der Anordnung gemäß Figur 3 stromauf des Vorlaufbehälters 90 angeordnete Meßzelle 22 auch stromab dieses Vorlaufbehälters vor oder auch nach der Konzentratpumpe 24 angeordnet wird.

In Figur 4 ist eine weitere Ausführungsform dargestellt, mit der der Füllgrad des als Beutel ausgebildeten Behälters 16 bestimmt werden kann. Es werden dabei wiederum die gleichen Bezugszeichen wie in Figuren 1 bis 3 verwendet.

Zur Bestimmung des Füllgrades wird eine Wegaufnehmereinrichtung 110 verwendet, die sich aus den Gerüstteilen 112 und 114 als starre Basisteile einer Druckanordnung 116, bestehend aus einer Druckplatte, die auf den als Beutel ausgebildeten Behälter 16 drückt und einer gegen das Gerüstteil 112 festgelegten Druckfeder sowie einer Meßzelle 118 zur Bestimmung der zurückgelegten Meßstrecke zusammensetzt. Diese Meßzelle 118 ist über die Leitung 120 mit der Steuereinheit 34 verbunden und gibt an diese das aufgenommene Wegsignal ab.

Die Ausführungsform gemäß Figur 4 wird folgendermaßen betrieben:
Die Druckeinrichtung 110 drückt zunächst den Beutel 16 zusammen, so daß hierdurch der Abstand zwischen der Druckplatte 116 und dem Gerüstteil 114 als Parameter für die im Beutel 16 befindlichen Massen angesehen werden kann. Demzufolge kann dieses Signal von der Steuereinheit 34 verarbeitet werden. Insbesondere kann der Leerzustand des Beutels 16 zunächst bestimmt werden, der hier nur mit dem pulverförmigen Mittel gefüllt ist. Desgleichen kann nach der ersten Fülloperation mit Wasser die Auslenkung der Druckplatte 116 bestimmt werden, was als Absolutwert am Ende der Mischoperation zurückbleibt, d.h. der Abstand der beiden Teile 114 und 116 entspricht im wesentlichen diesem Signal, so daß hierdurch die Steuereinheit 34 die Mischoperation beenden kann.

Andererseits kann jedoch aber auch - wie eingangs erläutert - die schrittweise Abnahme des Abstandes zwischen diesen beiden Teilen 114 und 116 bestimmt werden.

Anstelle des Wegaufnehmers 110 kann auch eine Wägeanordnung eingesetzt werden, an der der Beutel 16 aufgehängt wird. Auch hier kann das Anfangsgewicht ohne und mit Zuführung einer bestimmten Wassermenge ermittelt und in der Steuereinheit 34 gespeichert werden. Im übrigen wird auf die vorstehenden Erläuterungen verwiesen.

## Patentansprüche

1. Einrichtung zur Herstellung einer medizinischen Flüssigkeit, insbesondere Dialysierflüssigkeit, aus einem pulverförmigen Konzentrat (18) und Wasser, aufweisend eine Wasserquelle (12), eine erste Leitung (14) von der Wasserquelle zu einem das pulverförmige Konzentrat (18) enthaltenden Behälter (16), eine zweite Leitung (20), die sich von dem Behälter zu einem Mischpunkt (28) erstreckt, der in eine dritte, von der Wasserquelle abgehenden Leitung (26) gelegt ist, eine Einrichtung (24) zur Regelung des Flusses der Konzentratflüssigkeit, die in die zweite Leitung geschaltet ist, eine Flüssigkeitsfördereinrichtung (32) in der dritten Leitung stromab des Mischpunkts, eine erste Meßzelle im Leitungssystem, eine Steuereinheit (39), die auf das Signal der ersten Meßzelle hin die Einrichtung zur Regelung des Flusses der Konzentratflüssigkeit in vorbestimmter Weise derart steuert, daß die am Mischpunkt erzeugte Dialysierflüssigkeit einer vorbestimmten Zusammensetzung entspricht, sowie ein eine zweite Meßzelle (30) aufweisendes Schutzsystem, das stromab des Mischpunkts angeordnet ist und die Einrichtung bei falscher Zusammensetzung der Dialysierflüssigkeit abschaltet, dadurch gekennzeichnet, daß die erste Meßzelle (22) in die zweite Leitung (20) eingeschaltet ist.

2. Einrichtung nach Anspruch 1, dadurch gekennzeichnet, daß die erste und zweite Meßzelle (22, 30) jeweils eine Leitfähigkeitszelle ist.

3. Einrichtung nach Anspruch 1 oder 2, dadurch gekennzeichnet, daß eine Steuer- und Regeleinheit (34) mit der ersten Meßzelle (22) verbunden ist und deren Signal als Istwert mit einem Sollwert vergleicht und aufgrund des Vergleichsergebnisses die Konzentratpumpe (24) als Einrichtung zur Regelung des Flusses der Konzentratflüssigkeit regelt.

4. Einrichtung nach einem der Ansprüche 1 bis 3, dadurch gekennzeichnet, daß in der zweiten Leitung (20) stromauf der Konzentratpumpe (24) ein Vorlaufbehälter (90) angeordnet ist, an dem eine Niveausensoreinheit (92) angebracht ist, daß stromauf des Behälters (16) ein Einlaßventil (98) in die erste Leitung (14) und stromab des Behälters (16) ein Auslaßventil (100) in die zweite Leitung (20) eingeschaltet sind und daß auf das Signal der Niveausensoreinheit (92) hin die Regeleinheit (34) in vorbestimmter Weise das Einlaßventil (98) und das Auslaßventil (100) aktiviert bzw. desaktiviert.

5. Einrichtung nach einem der Ansprüche 1 bis 4, dadurch gekennzeichnet, daß der Behälter (16) als Beutel ausgebildet ist.

6. Einrichtung nach einem der Ansprüche 1 bis 5, dadurch gekennzeichnet, daß der Behälter (16) an einer Volumen-/Gewichtsmeßeinrichtung zur laufenden Bestimmung seines Volumens/Gewichts angebracht ist, die ihr Signal an die Steuer-/Regeleinheit (34) übermittelt.

7. Einrichtung nach Anspruch 6, dadurch gekennzeichnet, daß der beutelartige Behälter (16) in einer Wegaufnahmeeinrichtung (110) angeordnet ist.

## Claims

1. Apparatus for preparing a medicinal solution, in particular dialysis solution, from a pulverulent concentrate (18) and water, comprising a water source (12), a first conduit (14) from the water source to a container (16) containing the pulverulent concentrate (18), a second conduit (20) extending from the container to a mixing point (28) which is located in a third conduit (26) originating from the water source, a means (24) for controlling the flow of the concentrate solution connected into the second conduit, a solution conveying means (32) in the third conduit downstream of the mixing point, a first measuring cell in the conduit system, a control unit (39) controlling the means for controlling the flow of the concentrate solution in predetermined manner in response to the signal of the first measuring cell such that the dialysis solution generated at the mixing point corresponds to a predetermined composition, and a protective system which comprises a second measuring cell (30) and which is arranged downstream of the mixing point and switches off the apparatus in the event of an incorrect composition of the dialysis solution, characterized in that the first measuring cell (22) is connected into the second conduit (20).

2. Apparatus according to claim 1, characterized in that the first and second measuring cells (22, 30) are each a conductivity cell.

3. Apparatus according to claim 1 or 2, characterized in that a control and regulating unit (34) is connected to the first measuring cell (22) and compares the signal thereof as actual value with a desired value and on the basis of the result of the comparison controls the concentrate pump (24) as means for regulating the flow of concentrate solution.

4. Apparatus according to any one of claims 1 to 3, characterized in that in the second conduit (20) upstream of the concentrate pump (24) a preliminary container (90) is arranged to which a level sensor unit (92) is attached, that upstream of the container (16) an inlet valve (98) is connected into the first conduit (14) and downstream of the container (16) an outlet valve (100) is connected into the second conduit (20) and that in response to the signal of the level sensor unit (92) the control unit (34) activates or deactivates the inlet valve (98) and the outlet valve (100) in predetermined manner.

5. Apparatus according to any one of claims 1 to 4, characterized in that the container (16) is constructed as bag.

6. Apparatus according to any one of claims 1 to 5, characterized in that the container (16) is attached to a volume/weight measuring device for continous determination of its volume/weight, said device transmitting its signal to the control/ regulating unit (34).

7. Apparatus according to claim 6, characterized in that the bag-like container (16) is arranged in a displacement pickup means (110).

## Revendications

1. Dispositif pour la production d'un liquide médicinal, en particulier de liquide de dialyse, à partir d'un concentré pulvérulent (18) et d'eau, comportant une source d'eau (12), une première conduite (14) allant de la source d'eau à un récipient (16) contenant le concentré (18), une deuxième conduite (20), qui va du récipient à un point de mélange (28), qui est situé dans une troisième conduite (26) partant de la source d'eau, un dispositif (24) pour la régulation de l'écoulement du liquide de concentré, qui est monté dans la deuxième conduite, un dispositif de refoulement de liquide (32) dans la troisième conduite en aval du point de mélange, une première cellule de mesure dans le système de conduites, une unité de commande (34) qui commande le dispositif pour la régulation de l'écoulement du liquide de concentré sur la base du signal de la première cellule de mesure d'une manière prédéterminée telle que le liquide de dialyse obtenu au point de mélange corresponde à une composition prédéterminée, ainsi qu'un système de protection comportant une deuxième cellule de mesure (30), système qui est disposé en aval du point de mélange et qui met le dispositif hors circuit dans le cas d'une composition erronée du liquide de dialyse, caractérisé en ce que la première cellule de mesure (22) est branchée dans la deuxième conduite (20).

2. Dispositif suivant la revendication 1, caractérisé en ce que les première et deuxième cellules de mesure (22, 30) sont chacune une cellule de mesure de conductibilité.

3. Dispositif suivant la revendication 1 ou 2, caractérisé en ce qu'une unité de commande et de régulation (34) est reliée à la première cellule de mesure (22) et compare le signal de celle-ci, en tant que valeur effective, avec une valeur de consigne et, sur la base du résultat de la comparaison, règle la pompe de concentré (24) en tant que dispositif pour la régulation de l'écoulement du liquide de concentré.

4. Dispositif suivant l'une quelconque des revendications 1 à 3, caractérisé en ce que, dans la deuxième conduite (20) en amont de la pompe de concentré (24) est disposé un récipient intermédiaire (90) auquel est reliée une unité de détection de niveau (92), qu'en amont du récipient (16) une vanne d'entrée (98) est insérée dans la première conduite (14) et qu'en aval du récipient (16) une vanne de sortie (100) est insérée dans la deuxième conduite (20) et que l'unité de régulation (34) sur la base du signal de l'unité de détection de niveau (92) active ou désactive de manière prédéterminée la vanne d'entrée (98) et la vanne de sortie (100).

5. Dispositif suivant l'une quelconque des revendications 1 à 4, caractérisé en ce que le récipient (16) a la forme d'une poche.

6. Dispositif suivant l'une quelconque des revendications 1 à 5, caractérisé en ce que le récipient (16) est associé à un dispositif de mesure de volume/poids pour la détermination continue de son volume/poids, dispositif de mesure qui transmet son signal à l'unité de commande/régulation (34).

7. Dispositif suivant la revendication 6, caractérisé en ce que le récipient (16) en forme de poche est disposé dans un dispositif de détection de déplacement (110).
